(19) Europäisches Patentamt
European Patent Office
Office européen des brevets

(11) Publication number: **0 008 196**
**B1**

(12) **EUROPEAN PATENT SPECIFICATION**

(45) Date of publication of patent specification: **07.09.83**

(51) Int. Cl.³: **C 07 C 103/52, A 61 K 37/02**

(21) Application number: **79301542.1**

(22) Date of filing: **01.08.79**

(54) **Pentapeptides, processes for their preparation, intermediates used in such processes, use in pharmaceutical compositions and compositions containing them.**

(30) Priority: **08.08.78 US 932089**

(43) Date of publication of application:
**20.02.80 Bulletin 80/4**

(45) Publication of the grant of the patent:
**07.09.83 Bulletin 83/36**

(84) Designated Contracting States:
**AT BE CH DE FR IT LU NL SE**

(56) References cited:
**FR - A - 2 338 925**
**FR - A - 2 347 336**
**FR - A - 2 364 889**

(73) Proprietor: **AMERICAN HOME PRODUCTS CORPORATION**
**685, Third Avenue**
**New York, New York 10017 (US)**

(72) Inventor: **Garsky, Victor Michael**
**802, Hollow Road**
**Radnor Chester, Pennsylvania (US)**

(74) Representative: **Porter, Graham Ronald et al,**
**C/O John Wyeth & Brother Limited Huntercombe Lane South Taplow**
**Maidenhead Berkshire, SL6 0PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 008 196 B1

Courier Press, Leamington Spa, England.

# Pentapeptides, processes for their preparation, intermediates used in such processes, use in pharmaceutical compositions and compositions containing them

This invention relates to novel peptides, more particularly to pentapeptides possessing pharmacological activity, to processes for their preparation, to intermediates used in such processes and to pharmaceutical compositions containing them.

Enkephalin, a natural opiate receptor agonist in the brain, has been identified [see Hughes et al., Nature, *258*, 577 (1975)] as a mixture of two pentapeptides:

H—Tyr—Gly—Gly—Phe—Met—OH (methionine-enkephalin)

and

H—Tyr—Gly—Gly—Phe—Leu—OH (leucine-enkephalin).

Both peptides mimic the ability of morphine to block electrically evoked contractions of mouse vas deferens and guinea pig ileum, and both inhibit the stereospecific receptor binding of the opiate antagonist 3H-naloxone in brain homogenates.

It has been proposed that enkephalin receptors may be sites at which morphine-like drugs exert their analgesic activities, and that enkephalin may be the modulator or transmittor in brain systems for pain suppression or analgesia. It has been reported that methionine-enkephalin and leucine-enkephalin, when administered by injection in the brain ventricle in rats, induce a profound analgesia that is fully reversible by naloxone. [See Belluzzi et al., Nature, *260*, 625 (1976)]. The enkephalins are inactive when administered peripherally, however, and it is believed that the enkephalins are rapidly destroyed by blood enzymes and/or are poorly transported across the blood-brain barrier.

The amino acid sequence of methionine-enkephalin is identical to that of the N-terminal portion of the C-fragment ($\beta$-endorphin or $\alpha$-LPH[61—91]) of the peptide $\beta$-lipotropin, which is found in large concentrations in the pituitary and in much lower concentrations in the brain. Other naturally-occurring fragments of $\beta$-lipotropin are known, for example: $\alpha$-endorphin ($\beta$-LPH[61—76]) and $\gamma$-endorphin ($\beta$-LPH[61—77]). Both $\beta$-lipotropin and the endorphins show morphine-like properties in various test systems, and it has been suggested that methionine-enkephalin is a breakdown product of the large opiate-like peptides. Enkephalin, its relationship to $\beta$-lipotropin and the endorphins, and the pharmacological properties thereof, are reviewed in an article by Iverson et al., Nature, *262*, 738 (1976). Recent developments are also described in detail in the "Proceedings of the International Narcotics Research Club Meeting", Aberdeen, U.K. July 19—22, 1976, published in *Opiates and Endogenous Opioid Peptides*, North Holland Publishing Company, Amsterdam, 1976.

Various structural variations of methionine-enkephalin and leucine-enkephalin are described in the literature. For example, the pentapeptide

H—Tyr—Gly—Gly—Phe—Thr—OH,

wherein the fifth amino acid residue (methionine or leucine) is replaced by threonine, is described by Chang et al., Life Sciences, *18*, 1473 (1976). Similarly, a long-acting synthetic pentapeptide,

Tyr—D—Ala—Gly—Phe—Met-amide

is described in Pert et al., Science, *194*, 330 (1976); which compound, like the natural enkephalins, is inactive when administered peripherally. Baxter et al., British Journal of Pharmacology, March 2, 1977, pages 455P—456P and 523P report activity in the compound

Tyr—D—Ala—Gly—Phe—D—Leu

when administered intracerebroventricularly.

The present invention provides novel synthetic pentapeptides which have pharmacological activity, e.g. the ability to produce an analgesic effect in warm-blooded mammals.

This invention provides a chemical compound of formula:

$$\begin{array}{cccccccccccccccccccc} & & & & & & & & & & & & & & & & & CH_2OH & & \\ & & & & & & & & & & & & & & & & & | & & \\ R^1 & — & N & — & Tyr & — & N & — & D—Ser—Gly & — & N & — & Phe & — & N & — & D— & CH & — & X^2 \\ & & | & & & & | & & & & | & & & & | & & & & & \\ & & H & & & & R^2 & & & & R^3 & & & & R^4 & & & & & \end{array} \qquad (I)$$

wherein $R^1$ is hydrogen, methyl, ethyl, propyl, 2-methyl-2-pentenyl, 2-methyl-1-pentenyl, cyclopropylmethyl, or cyclobutylmethyl; $R^2$, $R^3$ and $R^4$ are, independently, hydrogen or methyl; and $X^2$ is COX

wherein X is —OH, —$NH_2$, —$NHC_nH_{2n+1}$ where n is 1, 2, 3 or 4 or —$OR^5$, where $R^5$ is lower alkyl of from 1 to 4 carbon atoms, or $X^2$ is $CH_2OR^6$ wherein $R^6$ is hydrogen or lower alkyl of 1 to 4 carbon atoms; or a pharmaceutically acceptable salt thereof.

Examples of lower alkyl groups are methyl, ethyl, propyl, isopropyl and n-butyl.

All chiral amino acids in the above formula, and throughout this specification and claims, are in the L- or natural configuration, unless otherwise indicated.

The tangible embodiments of the invention possess the inherent physical properties of being white to light tan coloured solids, are substantially insoluble in organic solvents such as chloroform, benzene, and the like, but exhibit solubility in water and aqueous acid solutions such as hydrochloric and acetic. These compounds display no clearly discernible melting points. Amino acid analyses of the compounds of the invention are consistent with the structures as set forth.

The tangible embodiments of the invention possess the inherent applied use characteristics of producing analgesia upon administration to warm-blooded animals, as evidenced by standard test procedures.

Preferably $R^1$ and $R^3$ are methyl. Preferably $R^2$ and $R^4$ are hydrogen. Preferably X is $NH_2$ or $NHC_nH_{2n+1}$.

Particularly preferred are compounds of the formula:

N—Me—Tyr—D—Ser—Gly—N—Me—Phe—D—Ser—X (Ia)

where X is $NH_2$ or $NHC_nH_{2n+1}$ where n is 1, 2, 3, or 4 or a pharmaceutically acceptable salt thereof.

This invention also provides protected intermediates for the pentapeptides of formula I including compounds having the formula:

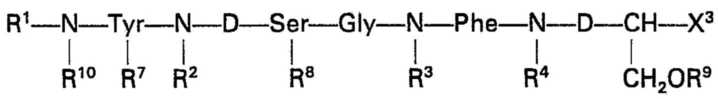

(II)

wherein $R^1$, $R^2$, $R^3$ and $R^4$ are as hereinbefore defined, $R^7$ represents a protecting group for the side chain hydroxyl of tyrosine, $R^8$ and $R^9$ represent protecting groups for the side chain hydroxyl of serine, $R^{10}$ is an $\alpha$-amino protecting group and $X^3$ represents $COX^1$ wherein $X^1$ is OH, $NH_2$, $NHC_nH_{2n+1}$, $OR^5$ wherein $R^5$ is as defined above, $OR^{11}$ wherein $R^{11}$ is a carboxyl protecting group other than $R^5$, or a solid phase resin support in which the bridging link between —CO— and a phenyl of the resin support has the formula

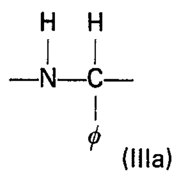

or —O—$CH_2$—

(IIIa) (IIIb)

wherein $\phi$ represents phenyl, or $X^3$ represents $CH_2OR^{12}$ wherein $R^{12}$ is lower alkyl of 1 to 4 carbon atoms, a hydroxyl protecting group as defined by $R^9$ or hydrogen.

Examples of $\alpha$-amino protecting groups for $R^{10}$

(1) acyl type protecting groups illustrated by the following: formyl, trifluoroacetyl, phthalyl, *p*-toluenesulfonyl (tosyl), nitrophenylsulfenyl, etc;

(2) aromatic urethane type protecting groups illustrated by benzyloxycarbonyl and substituted benzyloxycarbonyl such as *p*-chlorobenzyloxycarbonyl, *p*-nitrobenzyloxycarbonyl;

(3) aliphatic urethane protecting groups illustrated by *tert*-butyloxycarbonyl, diisopropylmethoxycarbonyl, Isopropyloxycarbonyl;

(4) cycloalkyl urethane type protecting groups illustrated by cyclopentyloxycarbonyl; adamantyloxycarbonyl, cyclohexyloxycarbonyl;

(5) thiourethane type protecting groups such as phenylthiocarbonyl;

(6) alkyl type protecting groups as illustrated by triphenylmethyl (trityl);

(7) trialkylsilane groups such as trimethylsilane. The preferred $\alpha$-amino protecting group is *tert*-butyloxycarbonyl.

Examples of $R^7$, $R^8$ and $R^9$ are independently tosyl, benzyloxycarbonyl, tetrahydropyran-2-yl, acetyl, benzoyl, *tert*-butyl, benzyl and dichlorobenzyl. Preferably $R^7$ is 2,6-dichlorobenzyl.

This invention also provides processes for preparing the compounds of the invention.

The compounds of formula I may be prepared by removing all the protecting groups and the resin

support when present from a protected precursor peptide, if desired converting a compound of formula I obtained to a pharmaceutically acceptable salt. Thus this invention provides a process for preparing a compound of formula I as hereinbefore defined which comprises removing the protecting groups and the resin support when present from a compound of formula:

```
R¹—N—Tyr—N—D—Ser—Gly—N—Phe—N—D—CH—X³        (II)
   |       |     |         |         |     |
   R¹⁰ R⁷  R²    R⁸        R³        R⁴    CH₂OR⁹
```

wherein $R^1$, $R^2$, $R^3$, $R^4$, $R^7$, $R^8$, $R^9$, $R^{10}$ and $X^3$ are as hereinbefore defined, and if desired converting a compound of formula I obtained to a pharmaceutically acceptable salt.

Removal of the protecting groups may be effected by methods known in the art for the respective protecting groups. Preferably the protecting groups are removed in a single step, for example, by using hydrogen fluoride preferably in the presence of anisole.

It will be apparent to those skilled in the art in the foregoing deprotection process the C-terminal lower alkyl ester function of a compound of formula II (i.e. wherein $X^1$ is $OR^5$ wherein $R^5$ is lower alkyl) may be converted to a carboxyl function when the process of deprotection involves hydrolysis. Accordingly it may be necessary to esterify a product having a terminal carboxy group to obtain a desired compound of formula I, wherein X is $OR^5$ wherein $R^5$ is lower alkyl of 1 to 4 carbon atoms.

Protecting groups and methods for removing them are well known in the art—see for example E. Schroder and K. Lubke "The Peptides" volume 1 Academic Press, New York and London, 1965.

The resin support when present may be cleaved at the same time as removal of the protecting groups, e.g. by using hydrogen fluoride, preferably in the presence of anisole. Alternatively the resin may be cleaved prior to deprotection. When the polystyrene resin support has a bridging link of formula IIIa cleaving produces a pentapeptide having a C-terminal $NH_2$ group. When the polystyrene resin support has a bridging link of formula IIIb cleaving by trans-esterification using a lower alkanol having 1 to 4 carbon atoms produces a peptide of formula II having a C-terminal lower alkyl ester group. However, cleaving by amidation using ammonia or an amine of formula

$$H_2NC_nH_{2n+1} \qquad (IV)$$

wherein n is as hereinbefore defined gives a peptide of formula II having a C-terminal amide function (i.e. $CONH_2$ or $CONHC_nH_{2n+1}$). Cleaving by trans-esterification, e.g. using dimethylaminoethanol, followed by mild hydrolysis in the manner described by Barton et al., J. Am. Chem. Soc, *95* 4501—6 1973, produces a peptide of formula II having a terminal carboxyl group (i.e. $X^1$=OH).

A compound of formula II wherein $X^1$ is OH may be converted to a compound of formula II wherein $R^{12}$ is hydrogen by reducing the terminal carboxy group using a reducing agent such as diborane. The product may be alkylated e.g. using an alkyl halide and base to give a compound of formula II wherein $R^{12}$ is lower alkyl having 1 to 4 carbon atoms.

The compounds of this invention may be built up by classical or solid phase synthesis. Typical solid phase procedures utilize either a benzhydrylamine resin or a chloromethylated polystyrene resin. Cleavage from the resin may be effected by HF. The pentapeptide can be purified by partition chromatography on Sephadex G-10 using 0.2 N acetic acid as the elutant. The selection of an appropriate resin support and elutant systems is well within the skill of the art.

Hence this invention also provides a process for preparing a compound of formula II wherein $X^1$ represents a solid phase resin support which comprises sequentially coupling the requisite, suitably protected and/or activated, amino acids under solid phase synthesis conditions to a benzhydrylamine, chloromethylated or hydroxymethyl polystyrene resin.

In the solid phase method as applied to the compounds of this invention, the $\alpha$-amino protected amino acid

```
D—Ser
   |
   R⁹
```

for example t-Boc-O-benzyl-D-serine, is first attached to a polystyrene resin and then the $\alpha$-amino protecting group is removed. Standard cleaving reagents and conditions for removal of specific $\alpha$-amino protecting groups may be used as described in Schroder and Lubke, "The Peptides", *1*, 72—75 (Academic Press, 1965). After removal of the $\alpha$-amino protecting group, the next desired protected amino acids are coupled individually to the resin supported sequence, *seriatim*. Alternatively, small peptide fragments may be prepared by, for example, the solution method and introduced into the solid phase reactor in the desired order. Each protected amino acid or amino acid sequence is introduced into the solid phase reactor in about a four fold excess. The coupling is conveniently carried out in dimethylformamide, methylene chloride, or a mixture of two solvents. The success of each coupling

reaction at each stage of the synthesis is determined by the ninhydrin reaction as described by E. Kaiser et al., Analyt. Biochem., *34*, 595 (1970). Where incomplete coupling has occurred, the reaction is repeated before the $\alpha$-amino protecting group is removed for introduction of the next amino acid or amino acid sequence.

The preferred coupling reagents are 1-hydroxybenzotriazole and diisopropylcarbodiimide; other such reagents will be familiar to those skilled in the art.

After the desired amino acid sequence has been synthesized, the polypeptide is removed from the resin support by treatment with, for example, hydrogen fluoride and anisole to obtain the fully deprotected linear polypeptide, wherein X is $NH_2$ or OH depending on the bridging link as described above.

Protecting groups well known in the art can be employed throughout the solid phase synthesis. The $\alpha$-amino protecting groups employed with each amino acid introduced in sequence of the ultimate polypeptide are of the

(1) acyl type protecting groups illustrated by the following: formyl, trifluoroacetyl, phthalyl, *p*-toluenesulfonyl (tosyl), nitrophenylsulfenyl, etc.
(2) aromatic urethane type protecting groups illustrated by benzyloxycarbonyl and substituted benzyloxycarbonyl such as *p*-chlorobenzyloxycarbonyl, *p*-nitrobenzyloxycarbonyl;
(3) aliphatic urethane protecting groups illustrated by *tert*-butyloxycarbonyl, diisopropylmethoxycarbonyl, isopropyloxycarbonyl, allyloxycarbonyl, 2,2,2-trichloroethoxycarbonyl, amyloxycarbonyl;
(4) cycloalkyl urethane type protecting groups illustrated by cyclopentyloxycarbonyl, adamantyloxycarbonyl, cyclohexyloxycarbonyl;
(5) thiourethane type protecting groups such as phenylthiocarbonyl;
(6) alkyl type protecting groups as illustrated by triphenylmethyl (trityl);
(7) trialkylsilane groups such as trimethylsilane. The preferred $\alpha$-amino protecting group is *tert*-butyloxycarbonyl.

In the classical method as applied to the compounds of formula II the desired peptide is built up by condensing amino acids or their derivatives or groups of amino acids which are protected if necessary. The C-terminal amino acid, D-serine, is protected and may be in the form of an amide, lower alkylamide, lower alkyl ester derivative of 1 to 4 carbon atoms, or in reduced form

(i.e. —N—$CH(CH_2OR^9)CH_2OR^{12}$)
|
$R^4$

to give the desired terminal group in the final product.

The condensation reactions may be carried out using methods generally known to form amide bonds in peptide and penicillin chemistry. To promote facile condensation of the amino acids it is preferred to employ a condensing agent. Examples of condensing agents are carbodiimides; e.g. N,N'-dicyclohexylcarbodiimide, (DCC), N,N'-diisopropylcarbodiimide. Alternatively the condensation may be effected by activating one or both of the terminal groups. Examples of the activated form of the terminal carboxyl are the acid chloride, anhydride, azide and the activated ester. The condensation reactions should be compatible with the protecting group(s) on the amino acids.

Accordingly, this invention provides a process for preparing a compound of formula II wherein $X^3$ is $COX^1$ wherein $X^1$ is $NH_2$, $NHC_nH_{2n+1}$, $OR^5$ and $OR^{11}$, or $X^3$ is $CH_2$ $OR^{12}$ wherein $R^{12}$ is other than hydrogen which comprises coupling the requisite amino acids and

D—$HNCH(CH_2OR^9)X^3$
|
$R^4$

wherein $X^3$ is as defined above each, suitably protected and/or activated, to give the desired sequence.

Methods of activating amino acids prior to coupling and coupling methods themselves are well known in the art—see for example the textbook of Schroder and Lubke mentioned above.

In selecting a particular side chain protecting group to be used in the synthesis of the peptides of the invention, the following rules should be followed: (a) any side chain protecting group must be stable to the reagent and under the reaction conditions selected for removing the $\alpha$-amino protecting group at each step of the synthesis (b) the protecting group must retain its protecting properties (i.e. not be split off under coupling conditions), and (c) the side chain protecting group must be removable upon the completion of the synthesis containing the desired amino acid sequence under reaction conditions that will not alter the peptide chain.

Where a chloromethylated resin support is employed and the peptide is cleaved from the resin as the C-terminal carboxylic acid, salts of alkali metals, ammonium salts and primary lower alkyl amides are also produced by methods known to the art. Cleavage from the peptide-resin with a lower alkyl amine such as ethylamine yields the corresponding lower alkylamide.

The symbols used for representing the amino acid residues in formula I and in other formulae employed herein are defined according to the IUPAC-IUB Commission on Biochemical Nomenclature Recommendations (1971), *Archives of Biochemistry and Biophysics, 150,* 1—8 (1972). All chiral amino acid residues identified without prefix in this specification and in the claims are in the natural L-configuration. Amino acid residues preceded with the prefix "D—" are in the D, or "unnatural" configuration.

The pharmaceutically acceptable salts of the polypeptides of this invention are acid addition salts of the free base in which the acid may be either organic or inorganic, as for example, hydrochloric, phosphoric, maleic, acetic, citric, succinic, malic, and similar acids. Likewise, salts of the free peptidic acid are embraced by the expression "pharmaceutically acceptable salts", and include the sodium, potassium, ammonium, and lower alkylamine salts. The salts are prepared and isolated by conventional methods.

The analgesic activity of the polypeptides of this invention may be demonstrated by the rat-tail flick method of D'Amour and Smith, J. Pharmacol. Exp. Ther., *72,* 74 (1941). In this procedure, a representative polypeptide of this invention,

N—Me—Tyr—D—Ser—Gly—N—Me—Phe—D—Ser—$NH_2$,

acetate, produced significant analgesia in rats upon administration at a dose as low as 0.1 mg/kg. intravenously, and 0.5 mg/kg. subcutaneously.

In employing the compounds of the invention, the particular dose to be administered will vary somewhat depending on the degree of analgesia desired, the particular animal being treated, and the particular compound of the invention being employed. In general, an intravenous dose of from 0.05 to 2.5 mg/kg. or a subcutaneous dose of from 0.5 to 10 mg/kg. will produce the desired effect. Preferably, analgesic therapy is initiated by administering a low dose of compound, the dosage thereafter being increased in succeeding administrations until the desired degree of analgesia is obtained. The precise dose for production of a desired effect will be readily determined by one skilled in the art.

This invention also provides a pharmaceutical composition comprising a compound of formula I as hereinbefore defined or a pharmaceutically acceptable salt thereof, in conjunction with a pharmaceutically acceptable carrier.

The following examples for purposes of illustration, describe in detail the synthesis of particular compounds of the invention. The other compounds of the invention may be made in similar fashion by substituting the desired blocked amino acid at the appropriate step of the process.

Example 1

N-Methyl-Tyrosyl-D-Serylglycyl-N-Methyl-Phenylalanyl-D-Serinamide

(a) To a 200 ml. reaction vessel was added 10.0 g. of benzhydrylamine resin (9 m moles free amine content). The resin was then treated in the following manner:

1. methylene chloride (three times).
2. 5 minute prewash with 30% trifluoroacetic acid-methylene chloride (v/v) containing 0.5% dithioerythritol.
3. 25 minute treatment with the above-described trifluoroacetic acid.
4. methylene chloride (three times).
5. 10 minute treatment with triethylaminedimethylformamide (v/v).
6. dimethylformamide (three times).
7. methylene chloride (three times).

A contact time of 2 minutes was allowed for each wash unless otherwise indicated.

The resin was gently stirred with *t*-Boc-O-Bzl-D-Ser and 1-hydroxybenzotriazole (HOBT) in 50% methylene chloride-dimethylformamide (20 m moles *t* - Boc - O - Bzl - D - Ser and 22 m moles HOBT). Following the addition of the above reagents, the mixture was treated with 22 mmoles of diisopropylcarbodiimide (the DIC was added in two equal portions over 30 minutes). After stirring overnight, the peptide-resin was washed successively with dimethylformamide (twice), 12% triethylamine-dimethylformamide (once) and methylene chloride (thrice). To test for completeness of reaction, the peptide-resin was subjected to a ninhydrin colour test following the procedure of E. Kaiser et al., Analytical Chemistry, *34*, 595 (1970).

The deprotection of the attached amino acid was carried out as described in steps (2) through (7) above.

The following amino acid residues were then introduced consecutively: *t*-Boc-N-Me-Phe (20 mmoles, 22 mmoles HOBT and 22 mmoles DIC), *t*-Boc-glycine (20 mmoles, 22 mmoles HOBT and 22 mmoles DIC), *t*-Boc-O-Bzl-D-Ser (20 mmoles, 22 mmoles HOBT and 22 mmoles DIC), *t*-Boc-N-Me-O-2,6-Cl-Bzl-Tyr (20 mmoles, 22 mmoles HOBT and 22 mmoles DIC). The washed pentapeptide resin was dried *in vacuo* to yield 13.4 g of t - Butoxycarbonyl - N - methyl - O - 2,6 - dichlorobenzyl -

tyrosyl - O - benzyl - D - Seryl - Glycyl - N - methyl - phenylalanyl - O - benzyl - D - seryl - benzhydrylamine resin.

(b) The above-described pentapeptide resin (13.4 g.) was treated *in vacuo* with anhydrous liquid hydrogen fluoride (100 ml.) and anisole (10 ml.) at 0° for 1 hour. The hydrogen fluoride and anisole were removed under reduced pressure and the residue suspended in 2N acetic acid, filtered and the filtrate lyophilized to yield the above title product, 2.91 g., as the acetate salt.

The crude product was treated with AG-3-X4A ion exchange resin (acetate form) for 30 minutes, filtered and lyophilized 2.65 g.

Example 2

Purification and characterization of N-Methyl-Tyrosyl-D-Serylglycyl-N-Methyl-Phenylalanyl-D-Serinamide Acetate

The above-titled crude product was purified as follows: 1.3 g. of material is dissolved in a minimum amount of 2N acetic acid and applied to a column (2.5×200 cm.) of Sephadex G-10 in 2N acetic acid. The column was eluted with 2N acetic acid and 15 ml. fractions collected. The column effluent was monitored at 254 nm. Fractions 29—40 were combined and lyophilized to yield 0.979 g. The product (0.979 g.) was further purified by applying the material in a small volume of upper phase B:A:W, 4:1:5 (n-butanol:acetic acid:water) onto a column (2.5×150 cm.) of Sephadex G-25 medium previously equilibrated with lower phase of the above system and then upper phase. The column was eluted with upper phase B:A:W and 11 ml. fractions collected. The effluent was monitored as above. Tubes 79—90 were shown to be homogeneous by thin layer chromatography systems 4:1:5, $R_f$=0.28 and 7:7:6 (isoamyl alcohol:pyridine:water); $R_f$=0.66 on silica gel. Thin layer chromatograms were visualized with ninhydrin and chlorine peptide reagent. Amino acid analysis following methane sulfonic acid hydrolysis gave the following ratios: Ser 1.95; Gly 1.00.

Example 3

N-Methyl-Tyrosyl-D-Seryl-Glycyl-N-Methyl-Phenylalanyl-D-Serine

(a) Chloromethylated polystyrene resin is esterified with *t* - Boc - O - Benzyl - D - Serine according to the procedure of Gisin, Helv. Chim. Acta., *56*, 1976 (1973), and the resulting polymeric ester is treated according to the procedure of Example 1 for incorporation of *t* - Boc - N - Me - Phe, *t* - Boc - Gly, *t* - Boc - O - Bzl - D - Ser, and *t* - Boc - N - Me - O - 2,6 - Cl - Bzl - Tyr to give t - Butoxycarbonyl - N - methyl - O - 2,6 - dichlorobenzyl - tyrosyl - O - benzyl - D - seryl - Glycyl - N - methyl - phenylalanyl - O - benzyl - D - seryloxymethyl polystyrene resin;

(b) The resulting peptide resin is treated with HF as in Example 1, and the thus obtained title compound then purified and characterized according to the procedure of Example 2.

Example 4

N-Methyl-Tyrosyl-D-Seryl-Glycyl-N-Methyl-Phenylalanyl-D-Serine-Ethylamide

(a) Treatment of the peptido resin of Example 3 with ethylamine in a sealed flask for 10 hours followed by removal of excess ethylamine gives t - Butoxycarbonyl - N - methyl - O - 2,6 - dichlorobenzyl - tyrosyl - O - benzyl - D - seryl - glycyl - N - methyl - phenylalanyl - O - benzyl - D - serineethylamide.

(b) Treatment with HF as in Example 1 and extraction with dimethylformamide, filtration, and evaporation of the filtrate yields the title compound.

Example 5

When N - Me - Tyr - D - Ser - Gly - N - Me - Phe - D - Ser - $NH_2$, acetate was tested in the rat-tail flick procedure of D'Amour and Smith (*supra*), the following results were obtained:

| Dose mg/kg. | Route | No. showing analgesia/no. tested (minutes) | | | | |
|---|---|---|---|---|---|---|
| | | 15 | 30 | 60 | 90 | 120 |
| 5.0 | i.v. | 4/4 | 3/3 | 3/3 | (3/6 dead) | |
| 1.0 | i.v. | 6/6 | 6/6 | 6/6 | | |
| 0.1 | i.v. | 3/6 | 5/6 | 5/6 | | |
| 0.5 | s.c. | | 2/6 | 2/6 | 1/6 | 1/6 |
| 1.0 | s.c. | | 6/6 | 6/6 | 6/6 | 5/6 |

**Claims for the contracting states: BE, CH, DE, FR, IT, LU, NL, SE**

1. A compound of the general formula:

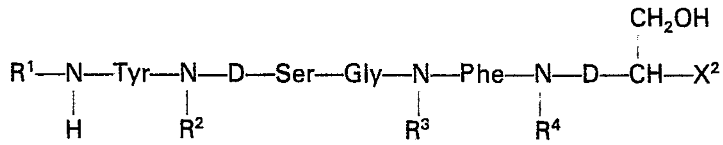 (I)

wherein $R^1$ is hydrogen, methyl, ethyl, propyl, 2-methyl-2-pentenyl, 2-methyl-1-pentenyl, cyclopropylmethyl, or cyclobutylmethyl; $R^2$, $R^3$ and $R^4$ are, independently, hydrogen or methyl; and $X^2$ is —COX wherein X is —OH, —$NH_2$, —$NHC_nH_{2n+1}$ where n is 1, 2, 3 or 4 or —$OR^5$, where $R^5$ is lower alkyl of from 1 to 4 carbon atoms, or $X^2$ is $CH_2OR^6$ wherein $R^6$ is hydrogen or lower alkyl of 1 to 4 carbon atoms; or a pharmaceutically acceptable salt thereof.

2. A compound of formula I as claimed in Claim 1 wherein $R^1$ is methyl, $R^2$ is hydrogen, $R^3$ is methyl and $R^4$ is hydrogen.

3. A compound of formula I as claimed in Claim 1 or Claim 2 wherein $X^2$ is COX wherein X is $NH_2$ or $NHC_nH_{2n+1}$.

4. A compound of formula I as defined in Claim 1 which is N - methyl - tyrosyl - D - seryl - glycyl - N - methyl - phenylalanyl - D - serinamide; N - methyl - tyrosyl - D - seryl - glycyl - N - methyl - phenylalanyl - D - serineethylamide or N - methyl - tyrosyl - D - seryl - glycyl - N - methyl - phenylalanyl - D - serine.

5. A compound as claimed in any one of Claims 1 to 4 when in the form of a hydrochloric, phosphoric, maleic, acetic, citric, succinic or malic acid addition salt or a sodium, potassium, ammonium or lower alkylamine salt.

6. A compound of the general formula:

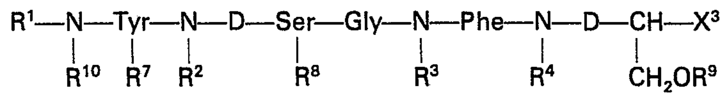 (II)

wherein $R^1$, $R^2$, $R^3$ and $R^4$ are as defined in Claim 1, $R^7$ represents a protecting group for the side chain hydroxyl of tyrosine, $R^8$ and $R^9$ represent protecting groups for the side chain hydroxyl of serine, $R^{10}$ is an $\alpha$-amino protecting group and $X^3$ represents $COX^1$ wherein $X^1$ is OH, $NH_2$, $NHC_nH_{2n+1}$ wherein n is 1, 2, 3 or 4, $OR^5$ wherein $R^5$ is as defined in Claim 1, $OR^{11}$ wherein $R^{11}$ is a carboxyl protecting group other than $R^5$, or a solid phase resin support in which the bridging link between CO and a phenyl of the resin support has the formula:

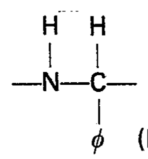 (IIIa) or —O—$CH_2$— (IIIb)

wherein $\phi$ represents phenyl or $X^3$ represents $CH_2OR^{12}$ wherein $R^{12}$ is lower alkyl of 1 to 4 carbon atoms, a hydroxyl protecting group as defined by $R^9$ or hydrogen.

7. A compound as claimed in Claim 6 wherein the $\alpha$-amino protecting group $R^{10}$ is selected from formyl, trifluoroacetyl, phthalyl, *p*-toluenesulfonyl, nitrophenylsulfenyl, benzyloxycarbonyl, *p*-chlorobenzyloxycarbonyl, *p*-nitrobenzyloxycarbonyl, *tert*-butyloxycarbonyl, diisopropylmethoxycarbonyl, isopropyloxycarbonyl, cyclopentyloxycarbonyl, adamantyloxycarbonyl, cyclohexyloxycarbonyl, phenylthiocarbonyl, triphenylmethyl and trimethylsilyl.

8. A compound as claimed in Claim 6 or Claim 7 wherein $R^7$, $R^8$ and $R^9$ are independently selected from tosyl, benzyloxycarbonyl, tetrahydropyran-2-yl, acetyl, benzoyl, *tert*-butyl, benzyl and dichlorobenzyl.

9. A compound of formula II as defined in Claim 6 which is t - butoxycarbonyl - N - methyl - O - 2,6 - dichlorobenzyl - tyrosyl - O - benzyl - D - seryl - glycyl - N - methyl - phenylalanyl - O - benzyl - D - seryl - benzhydrylamine resin; t - butoxy - carbonyl - N - methyl - O - 2,6 - dichlorobenzyl - tyrosyl - O - benzyl - D - seryl - glycyl - N - methyl - phenylalanyl - O - benzyl - D - seryloxymethyl polystyrene resin or t - butoxycarbonyl - N - methyl - O - 2,6 - dichlorobenzyl - tyrosyl - O - benzyl - D - seryl - glycyl - N - methyl - phenylalanyl - O - benzyl - D - serineethylamide.

10. A process for preparing a compound of formula

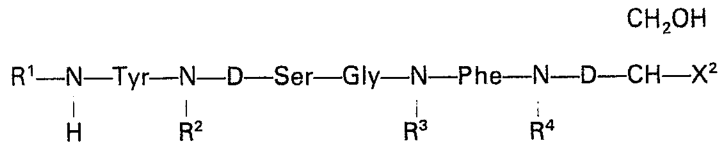

(I)

as defined in Claim 1 which comprises removing the protecting groups and cleaving the resin support when present from a compound of formula

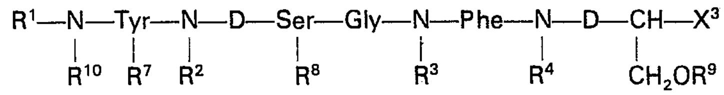

(II)

as defined in Claim 6, and if desired converting the compound of formula I obtained to a pharmaceutically acceptable salt.

11. A process as claimed in Claim 10 which is effected with hydrogen fluoride.

12. A process as claimed in Claim 10 or Claim 11 wherein $R^{10}$ is as defined in Claim 7 and $R^7$, $R^8$ and/or $R^9$ are/is as defined in Claim 8.

13. A process for preparing a compound of formula II as defined in Claim 6 which comprises:

(a) reducing a compound of formula II wherein $X^3$ is $COX^1$ wherein $X^1$ is OH to give a compound of formula II wherein $R^{12}$ is hydrogen and if desired alkylating the product to give a compound of formula II wherein $R^{12}$ is lower alkyl of 1 to 4 carbon atoms;

(b) sequentially coupling the requisite, suitably protected and/or activated, amino acids under solid phase synthesis conditions to a benzhydrylamine, chloromethylated or hydroxymethyl polystyrene resin to give a compound of formula II wherein $X^1$ is a solid phase polystyrene resin support, or

(c) coupling the requisite amino acids and

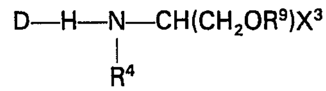

wherein $R^4$ and $R^9$ are as defined in Claim 6, and $X^3$ is $COX^1$, wherein $X^1$ is $NH_2$, $NHC_nH_{2n+1}$, $OR^5$ or $OR^{11}$, or $X^3$ is $CH_2OR^{12}$ wherein $R^{12}$ is other than hydrogen, each suitably protected and/or activated to give a corresponding compound of formula II wherein $X^3$ is as defined immediately above.

14. A process as claimed in Claim 10 in which the compound prepared is N - methyl - tyrosyl - D - seryl - glycyl - N - methylphenylalanyl - D - serinamide; N - methyl - tyrosyl - D - seryl - glycyl - N - methyl - phenylalanyl - D - serineethylamide or N - methyl - tyrosyl - D - seryl - glycyl - N - methyl - phenylalanyl - D - serine.

15. A process as claimed in Claim 13 in which the compound prepared is t - butoxycarbonyl - N - methyl - O - 2,6 - dichlorobenzyl - tyrosyl - O - benzyl - D - seryl - glycyl - N - methylphenylalanyl - O - benzyl - D - seryl - benzhydrylamine resin; t - butoxy - carbonyl - N - methyl - O - 2,6 - dichlorobenzyl - tyrosyl - O - benzyl - D - seryl - glycyl - N - methyl - phenylalanyl - O - benzyl - D - seryloxymethyl polystyrene resin or t - butoxycarbonyl - N - methyl - O - 2,6 - dichlorobenzyl - tyrosyl - O - benzyl - D - seryl - glycyl - N - methyl - phenylalanyl - O - benzyl - D - serineethylamide.

16. A pharmaceutical composition comprising a compound of formula I as defined in any one of Claims 1 to 4 or a pharmaceutically acceptable salt thereof, in conjunction with a pharmaceutically acceptable carrier.

17. A compound of formula I as claimed in any one of Claims 1 to 4 or a pharmaceutically acceptable salt thereof for use as a pharmaceutical.

**Claims for the contracting state AT**

1. A process for preparing a compound of the general formula:

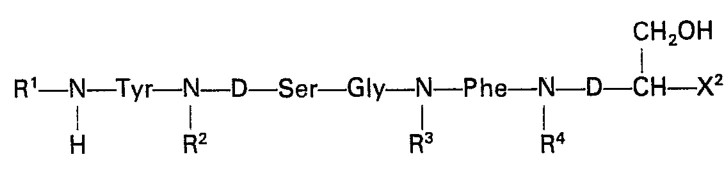

(I)

wherein $R^1$ is hydrogen, methyl, ethyl, propyl, 2-methyl-2-pentenyl, 2-methyl-1-pentenyl, cyclopropyl-

methyl, or cyclobutylmethyl; $R^2$, $R^3$ and $R^4$ are, independently, hydrogen or methyl; and $X^2$ is —COX wherein X is —OH, —$NH_2$, —$NHC_nH_{2n+1}$ where n is 1, 2, 3 or 4 or —$OR^5$, where $R^5$ is lower alkyl of from 1 to 4 carbon atoms, or $X^2$ is $CH_2OR^6$ wherein $R^6$ is hydrogen or lower alkyl of 1 to 4 carbon atoms; or a pharmaceutically acceptable salt thereof, which comprises removing the protecting groups and cleaving the resin support when present from a compound of the general formula:

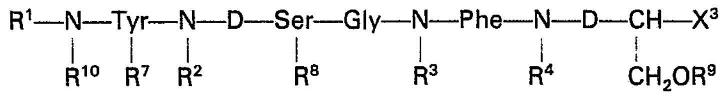

(II)

wherein $R^1$, $R^2$, $R^3$ and $R^4$ are as defined hereinabove, $R^7$ represents a protecting group for the side chain hydroxyl of tyrosine, $R^8$ and $R^9$ represent protecting groups for the side chain hydroxyl of serine, $R^{10}$ is an $\alpha$-amino protecting group and $X^3$ represents $COX^1$ wherein $X^1$ is OH, $NH_2$, $NHC_nH_{2n+1}$ wherein n is 1, 2, 3 or 4, $OR^5$ wherein $R^5$ is as defined hereinabove, $OR^{11}$ wherein $R^{11}$ is a carboxyl protecting group other than $R^5$, or a solid phase resin support in which the bridging link between CO and a phenyl of the resin support has the formula:

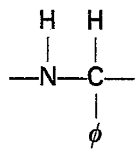

(IIIa) or —O—$CH_2$— (IIIb)

wherein $\phi$ represents phenyl or $X^3$ represents $CH_2OR^{12}$ wherein $R^{12}$ is lower alkyl of 1 to 4 carbon atoms, a hydroxyl protecting group as defined by $R^9$ or hydrogen, and if desired converting the compound of formula I obtained to a pharmaceutically acceptable salt.

2. A process as claimed in Claim 1 which is effected with hydrogen fluoride.

3. A process for preparing a compound of the general formula:

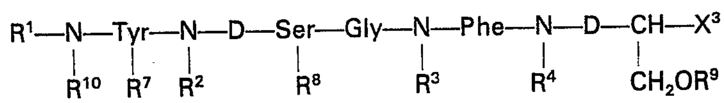

(II)

wherein $R^1$, $R^2$, $R^3$ and $R^4$ are as defined in Claim 1 $R^7$ represents a protecting group for the side chain hydroxyl of tyrosine, $R^8$ and $R^9$ represent protecting groups for the side chain hydroxyl of serine, $R^{10}$ is an $\alpha$-amino protecting group and $X^3$ represents $COX^1$ wherein $X^1$ is OH, $NH_2$, $NHC_nH_{2n+1}$ wherein n is 1, 2, 3 or 4, $OR^5$ wherein $R^5$ is as defined hereinabove, $OR^{11}$ wherein $R^{11}$ is a carboxyl protecting group other than $R^5$, or a solid phase resin support in which the bridging link between CO and a phenyl of the resin support has the formula:

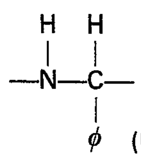

(IIIa) or —O—$CH_2$— (IIIb)

wherein $\phi$ represents phenyl or $X^3$ represents $CH_2OR^{12}$ wherein $R^{12}$ is lower alkyl of 1 to 4 carbon atoms, a hydroxyl protecting group as defined by $R^9$ or hydrogen, which comprises:

(a) reducing a compound of formula II wherein $X^3$ is $COX^1$ wherein $X^1$ is OH to give a compound of formula II wherein $R^{12}$ is hydrogen and if desired alkylating the product to give a compound of formula II wherein $R^{12}$ is lower alkyl of 1 to 4 carbon atoms; or

(b) sequentially coupling the requisite, suitably protected and/or activated, amino acids under solid phase synthesis conditions to a benzhydrylamine, chloromethylated or hydroxymethyl polystyrene resin to give a compound of formula II wherein $X^1$ is a solid phase polystyrene resin support, or

(c) coupling the requisite amino acids and

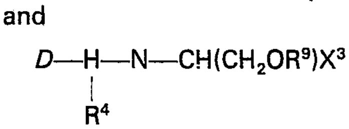

wherein $R^4$ and $R^9$ are as defined in Claim 1, and $X^3$ is $COX^1$, wherein $X^1$ is $NH_2$, $NHC_nH_{2n+1}$, $OR^5$ or $OR^{11}$, or $X^3$ is $CH_2OR^{12}$ wherein $R^{12}$ is other than hydrogen, each suitably protected and/or activated to give a corresponding compound of formula II wherein $X^3$ is as defined immediately above.

4. A process as claimed in any one of Claims 1 to 3 wherein the $\alpha$-amino protecting group $R^{10}$ is selected from formyl, trifluoroacetyl, phthalyl, *p*-toluenesulfonyl, nitrophenylsulfenyl, benzyloxycarbonyl, *p*-chlorobenzyloxycarbonyl, *p*-nitrobenzyloxycarbonyl, *tert*-butyloxycarbonyl, diisopropylmethoxycarbonyl, isopropyloxycarbonyl, cyclopentyloxycarbonyl, adamantyloxycarbonyl, cyclohexyloxycarbonyl, phenylthiocarbonyl, triphenylmethyl and trimethylsilyl.

5. A process as claimed in any one of Claims 1 to 4 wherein $R^7$, $R^8$ and $R^9$ are independently selected from tosyl, benzyloxycarbonyl, tetrahydropyran-2-yl, acetyl, benzoyl, *tert*-butyl, benzyl and dichlorobenzyl.

6. A process as claimed in Claim 1 or Claim 2 wherein $X^2$ is COX wherein X is $NH_2$ or $NHC_nH_{2n+1}$.

7. A process as claimed in any one of Claims 1 to 6 wherein $R^1$ is methyl, $R^2$ is hydrogen, $R^3$ is methyl and $R^4$ is hydrogen.

8. A process as claimed in Claim 1 in which the compound prepared is N - methyl - tyrosyl - D - seryl - glycyl - N - methyl - phenylalanyl - D - serinamide; N - methyl - tyrosyl - D - seryl - glycyl - N - methyl - phenylalanyl - D - serine - ethylamide or N - methyl - tyrosyl - D - seryl - glycyl - N - methyl - phenylalanyl - D - serine.

9. A process as claimed in Claim 1 or Claim 8 in which the compound of formula I prepared is in the form of a hydrochloric, phosphoric, maleic, acetic, citric, succinic or malic acid addition salt or a sodium, potassium, ammonium or lower alkylamine salt.

10. A process as claimed in Claim 3 in which the compound prepared is t - butoxycarbonyl - N - methyl - O - 2,6 - dichlorobenzyl - tyrosyl - O - benzyl - D - seryl - glycyl - N - methylphenylalanyl - O - benzyl - D - seryl - benzhydrylamine resin; t - butoxycarbonyl - N - methyl - O - 2,6 - dichlorobenzyl - tyrosyl - O - benzyl - D - seryl - glycyl - N - methyl - phenylalanyl - O - benzyl - D - seryloxymethyl polystyrene resin or t - butoxycarbonyl - N - methyl - O - 2,6 - dichlorobenzyl - tyrosyl - O - benzyl - D - seryl - glycyl - N - methyl - phenylalanyl - O - benzyl - D - serineethylamide.

**Revendications pour les états contractants BE, CH, DE, FR, IT, LU, NL, SE**

1. Composé de formule générale:

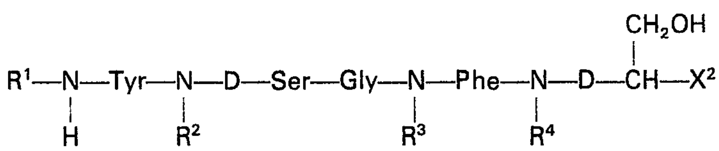

(I)

dans laquelle $R^1$ est l'hydrogène ou un groupe méthyle, éthyle, propyle, 2-méthyl-2-pentényle, 2-méthyl-1-pentényle, cyclopropylméthyle ou cyclobutylméthyle; $R^2$, $R^3$ et $R^4$ représentent, indépendamment, l'hydrogène ou un groupe méthyle; et $X^2$ est une groupe —COX dans lequel X est un radical —OH, —$NH_2$, —$NHC_nH_{2n+1}$ où *n* a la valeur 1, 2, 3 ou 4 ou —$OR^5$, où $R^5$ est un radical alkyle inférieur ayant 1 à 4 atomes de carbone, ou bien $X^2$ est un groupe $CH_2OR^6$ dans lequel $R^6$ est l'hydrogène ou un radical alkyle inférieur ayant 1 à 4 atomes de carbone; ou un sel pharmaceutiquement acceptable de ce composé.

2. Composé de formule I suivant la revendication 1, dans lequel $R^1$ est le groupe méthyle, $R^2$ est l'hydrogène, $R^3$ est le groupe méthyle et $R^4$ est l'hydrogène.

3. Composé de formule I suivant la revendication 1 ou la revendication 2, dans lequel $X^2$ représente COX, où X est un radical $NH_2$ ou $NHC_nH_{2n+1}$.

4. Composé de formule I suivant la revendication 1, qui est le N - méthyl - tyrosyl - D - séryl - glycyl - N - méthyl - phénylalanyl - D - sérinamide; le N - méthyl - tyrosyl - D - séryl - glycyl - N - méthyl - phénylalanyl - D - sérinéthylamide ou la N - méthyl - tyrosyl - D - séryl - glycyl - N - méthyl - phénylalanyl - D - sérine.

5. Composé suivant l'une quelconque des revendications 1 à 4 sous la forme d'un sel d'addition d'acide chlorhydrique, phosphorique, maléique, acétique, citrique, succinique ou malique ou d'un sel de sodium, de potassium, d'ammonium ou d'alkylamine inférieure.

6. Composé de formule générale:

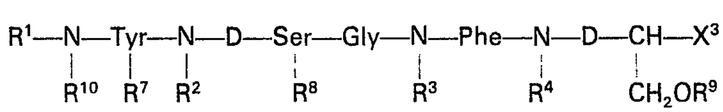

(II)

dans laquelle $R^1$, $R^2$, $R^3$ et $R^4$ ont les définitions données dans la revendication 1, $R^7$ représente un groupe protecteur pour le groupe hydroxyle en chaîne latérale de la tyrosine, $R^8$ et $R^9$ représentent des groupes protecteurs pour les groupes hydroxyle en chaîne latérale de la sérine, $R^{10}$ est un groupe protégeant la fonction $\alpha$-amino et $X^3$ représente un groupe de formule $COX^1$, dans laquelle $X^1$ est un

radical OH, $NH_2$, $NHC_nH_{2n+1}$, où *n* est égal à 1, 2, 3 ou 4, un groupe $OR^5$ dans lequel $R^5$ a la définition donnée dans la revendication 1, un groupe $OR^{11}$ dans lequel $R^{11}$ est un groupe protégeant la fonction carboxyle autre que $R^5$, ou un support formé d'une résine en phase solide dans lequel la liaison de pontage entre CO et un groupe phényle de la résine de support répond à la formule:

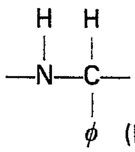 (IIIa) ou $-O-CH_2-$ (IIIb)

où $\phi$ représente le groupe phényle, ou bien $X^3$ représente un groupe $CH_2OR^{12}$ dans lequel $R^{12}$ est un radical alkyle inférieur ayant 1 à 4 atomes de carbone, un groupe protégeant la fonction hydroxyle comme défini par $R^9$ ou l'hydrogène.

7. Composé suivant la revendication 6, dans lequel le groupe $R^{10}$ protégeant la fonction $\alpha$-amino est choisi entre les groupes formyle, trifluoracétyle, phtalyle, p-toluènesulfonyle, nitrophénylsulfényle, benzyloxycarbonyle, *p*-chlorobenzyloxycarbonyle, *p*-nitrobenzyloxycarbonyle, tertio-butyloxycarbonyle, diisopropylméthoxycarbonyle, isopropyloxycarbonyle, cyclopentyloxycarbonyle, adamantyloxycarbonyle, cyclohexyloxycarbonyle, phénylthiocarbonyle, triphénylméthyle et triméthylsilyle.

8. Composé suivant la revendication 6 ou la revendication 7, dans lequel $R^7$, $R^8$ et $R^9$ sont choisis, indépendamment, entre les groupes tosyle, benzyloxycarbonyle, tétrahydropyranne-2-yle, acétyle, benzoyle, tertio-butyle, benzyle et dichlorobenzyle.

9. Composé de formule II tel que défini dans la revendication 6, qui est la tertio-butoxycarbonyl - N - méthyl - O - 2,6 - dichlorobenzyl - tyrosyl - O - benzyl - D - séryl - glycyl - N - méthyl - phénylalanyl - O - benzyl - D - séryl - benzhydrylamine - résine; la tertio - butoxycarbonyl - N - méthyl - O - 2,6 - dichlorobenzyl - tyrosyl - O - benzyl - D - séryl - glycyl - N - méthyl - phényl - alanyl - O - benzyl - D - séryloxyméthyl - polystyrène - résine ou le tertio - butoxycarbonyl - N - méthyl - O - 2,6 - dichlorobenzyl - tyrosyl - O - benzyl - D - séryl - glycyl - N - méthyl - phénylalanyl - O - benzyl - D - sérinéthylamide.

10. Procédé de préparation d'un composé de formule:

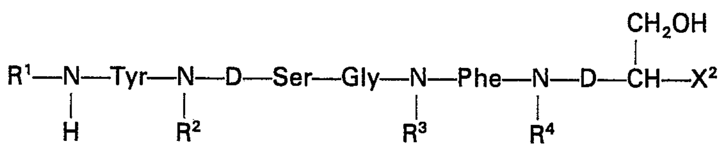 (I)

comme défini dans la revendication 1, qui consiste à éliminer les groupes protecteurs et à cliver le support de résine, lorsqu'il est présent, d'un composé de formule:

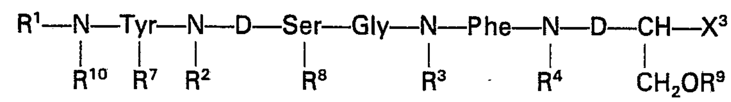 (II)

comme défini dans la revendication 6 et, le cas échéant, à convertir le composé de formule I obtenu en un sel pharmaceutiquement acceptable.

11. Procédé suivant la revendication 10, qui est mis en oeuvre avec le fluorure d'hydrogène.

12. Procédé suivant la revendication 10 ou la revendication 11, dans lequel $R^{10}$ a la définition donnée dans la revendication 7 et $R^7$, $R^8$ et/ou $R^9$ ont la définition donnée dans la revendication 8.

13. Procédé de préparation d'un composé de formule II comme défini dans la revendication 6, qui consiste:

(a) à réduire un composé de formule II dans laquelle $X^3$ est un groupe $COX^1$ dans lequel $X^1$ est un radical OH pour former un composé de formule II dans laquelle $R^{12}$ est l'hydrogène et, le cas échéant, à alkyler le produit pour obtenir un composé de formule II dans laquelle $R^{12}$ est un radical alkyle inférieur ayant 1 à 4 atomes de carbone;

(b) à combiner successivement les amino-acides désirés, convenablement protégés et/ou activés, dans des conditions de synthèse en phase solide en une résine de benzhydrylamine, de polystyrène chlorométhylé ou d'hydroxyméthylpolystyrène pour former un composé de formule II dans laquelle $X^1$ est un support formé d'une résine polystyrène en phase solide, ou bien

(c) à combiner les amino-acides désirés et un composé de formule

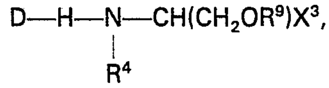

dans laquelle $R^4$ et $R^9$ ont la définition donnée dans la revendication 6, et $X^3$ est un groupe $COX^1$, dans lequel $X^1$ représente $NH_2$, $NHC_nH_{2n+1}$, $OR^5$ ou $OR^{11}$, ou bien $X^3$ est un groupe $CH_2OR^{12}$, dans lequel $R^{12}$ représente autre chose que de l'hydrogène, chacun étant convenablement protégé et/ou activé pour former un composé correspondant de formule II dans laquelle $X^3$ a la définition qui vient d'être donnée.

14. Procédé suivant la revendication 10, dans lequel le composé préparé est le N - méthyl - tyrosyl - D - séryl - glycyl - N - méthyl - phénylalanyl - D - sérinamide; le N - méthyl - tyrosyl - D - séryl - glycyl - N - méthyl - phénylalanyl - D - sérinéthylamide ou la N - méthyl - tyrosyl - D - séryl - glycyl - N - méthyl - phénylalanyl - D - sérine.

15. Procédé suivant la revendication 13, dans lequel le composé préparé consiste en tertio - butoxycarbonyl - N - méthyl - O - 2,6 - dichlorobenzyl - tyrosyl - O - benzyl - D - séryl - glycyl - N - méthyl - phénylalanyl - O - benzyl - D - séryl - résine de benzhydrylamine; en tertio - butoxycarbonyl - N - méthyl - O - 2,6 - dichlorobenzyl - tyrosyl - O - benzyl - D - séryl - glycyl - N - méthyl - phénylalanyl - O - benzyl - D - séryloxyméthyl - polystyrène - résine ou en tertio - butoxycarbonyl - N - méthyl - O - 2,6 - dichlorobenzyl - tyrosyl - O - benzyl - D - séryl - glycyl - N - méthyl - phénylalanyl - O - benzyl - D - sérinéthylamide.

16. Composition pharmaceutique comprenant un composé de formule I suivant l'une quelconque des revendications 1 à 4 ou un sel pharmaceutiquement acceptable de ce composé, conjointement avec un support pharmaceutiquement acceptable.

17. Un composé de formule I suivant l'une quelconque des revendications 1 à 4 ou un sel pharmaceutiquement acceptable de ce composé, destiné à être utilisé comme substance pharmaceutique.

**Revendications pour l'etat contractant AT**

1. Procédé de préparation d'un composé de formule générale:

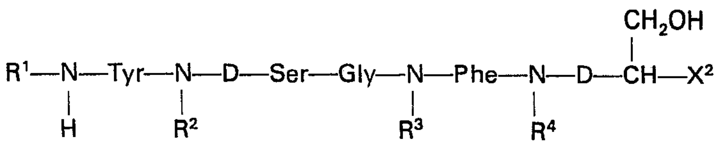

(I)

dans laquelle $R^1$ est l'hydrogène ou un radical méthyle, éthyle, propyle, 2-méthyl-2-penténylе, 2-méthyl-1-penténylе, cyclopropylméthyle ou cyclobutylméthyle; $R^2$, $R^3$ et $R^4$ représentent, indépendamment, l'hydrogène ou un groupe méthyle; et $X^2$ est un groupe —COX dans lequel X est un radical —OH, —$NH_2$, —$NHC_nH_{2n+1}$ où $n$ est égal à 1, 2, 3 ou 4 ou un groupe —$OR^5$ dans lequel $R^5$ est un radical alkyle inférieur ayant 1 à 4 atomes de carbone, ou bien $X^2$ est un groupe $CH_2OR^6$ dans lequel $R^6$ est l'hydrogène ou un radical alkyle inférieur ayant 1 à 4 atomes de carbone; ou d'un sel pharmaceutiquement acceptable de ce composé, qui consiste à éliminer les groupes protecteurs et à cliver la résine de support, lorsqu'elle est présente, d'un composé de formule générale:

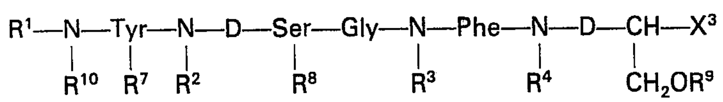

(II)

dans laquelle $R^1$, $R^2$, $R^3$ et $R^4$ ont la définition donnée ci-dessus, $R^7$ représente un groupe protecteur pour la fonction hydroxyle de la chaîne latérale de la tyrosine, $R^8$ et $R^9$ représentent des groupes protecteurs pour la fonction hydroxyle en chaîne latérale de la sérine, $R^{10}$ est un groupe protégeant la fonction $\alpha$-amino et $X^3$ représente un groupe $COX^1$ dans lequel $X^1$ est un radical OH, $NH_2$, $NHC_nH_{2n+1}$, où $n$ est égal à 1, 2, 3 ou 4, un groupe $OR^5$ dans lequel $R^5$ a la définition donnée ci-dessus, un groupe $OR^{11}$ dans lequel $R^{11}$ est un groupe protégeant la fonction carboxyle autre que le groupe $R^5$, ou une résine de support en phase solide, la liaison de pontage entre CO et un groupe phényle de la résine de support répondant à la formule:

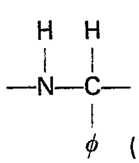

(IIIa)

ou

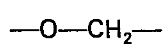

(IIIb)

où $\phi$ représente un groupe phényle ou bien $X^3$ représente un groupe $CH_2OR^{12}$ dans lequel $R^{12}$ est un

radical alkyle inférieur ayant 1 à 4 atomes de carbone, un groupe protégeant la fonction hydroxyle comme défini par $R^9$ ou l'hydrogène, et le cas échéant, à convertir le composé de formule I obtenu en un sel pharmaceutiquement acceptable.

2. Procédé suivant la revendication 1, qui est mis en oeuvre avec du fluorure d'hydrogène.

3. Procédé de préparation d'un composé de formule générale:

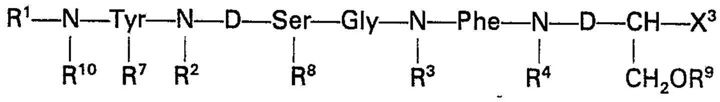

(II)

dans laquelle $R^1$, $R^2$, $R^3$ et $R^4$ ont les définitions données dans la revendication 1, $R^7$ représente un groupe protecteur pour la fonction hydroxyle en chaîne latérale de la tyrosine, $R^8$ et $R^9$ représentent des groupes protecteurs pour la fonction hydroxyle en chaîne latérale de la sérine, $R^{10}$ est un groupe protégeant la fonction $\alpha$-amino et $X^3$ représente un groupe $COX^1$ dans lequel $X^1$ est un radical OH, $NH_2$, $NHC_nH_{2n+1}$, où $n$ est égal à 1, 2, 3 ou 4, un groupe $OR^5$ dans lequel $R^5$ a la définition donnée ci-dessus, un groupe $OR^{11}$ dans lequel $R^{11}$ est un groupe protégeant la fonction carboxyle autre que le groupe $R^5$, ou une résine de support en phase solide, la liaison de pontage entre CO et un groupe phényle de la résine de support répondant à la formule:

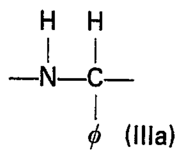

(IIIa) ou

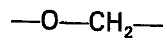

(IIIb)

où $\phi$ représente un groupe phényle, ou bien $X^3$ représente un groupe $CH_2OR^{12}$ dans lequel $R^{12}$ est un radical alkyle inférieur ayant 1 à 4 atomes de carbone, un groupe protégeant la fonction hydroxyle comme défini par $R^9$ ou l'hydrogène, procédé qui consiste:

(a) à réduire un composé de formule II dans laquelle $X^3$ est un groupe $COX^1$ dans lequel $X^1$ est OH, pour former un composé de formule II dans laquelle $R^{12}$ est l'hydrogène et, le cas échéant, à alkyler le produit pour obtenir un composé de formule II dans laquelle $R^{12}$ est un radical alkyle inférieur ayant 1 à 4 atomes de carbone; ou

(b) à combiner successivement les amino-acides désirés convenablement protégés et/ou activés, dans des conditions de synthèse en phase solide, en une résine de benzhydrylamine, une résine de polystyrène chlorométhylé ou une résine d'hydroxyméthyl-polystyrène pour obtenir un composé de formule II dans laquelle $X^1$ est un support formé d'une résine de polystyrène en phase solide, ou bien

(c) à combiner les amino-acides désirés et un composé

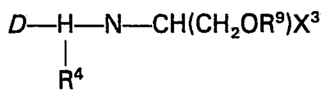

dans la formule duquel $R^4$ et $R^9$ ont les définitions données dans la revendication 1, et $X^3$ est un groupe de formule $COX^1$, dans laquelle $X^1$ est un radical $NH_2$, $NHC_nH_{2n+1}$, $OR^5$ ou $OR^{11}$, ou bien $X^3$ est un groupe $CH_2OR^{12}$, dans lequel $R^{12}$ représente autre chose que de l'hydrogène, chacun convenablement protégé et/ou activé pour former un composé correspondant de formule II dans laquelle $X^3$ a la définition donnée immédiatement ci-dessus.

4. Procédé suivant l'une quelconque des revendications 1 à 3, dans lequel le groupe $R^{10}$ protégeant la fonction $\alpha$-amino est choisi entre les groupes formyle, trifluoracétyle, phtalyle, p-toluènesulfonyle, nitrophénylsulfényle, benzyloxycarbonyle, *p*-chlorobenzyloxycarbonyle, *p*-nitrobenzyloxycarbonyle, tertio-butyloxycarbonyle, diisopropylméthoxycarbonyle, isopropyloxycarbonyle, cyclopentyloxycarbonyle, adamantyloxycarbonyle, cyclohexyloxycarbonyle, phénylthiocarbonyle, triphénylméthyle et triméthylsilyle.

5. Procédé suivant l'une quelconque des revendications 1 à 4, dans lequel $R^7$, $R^8$ et $R^9$ sont choisis indépendamment entre les radicaux tosyle, benzyloxycarbonyle, tétrahydropyranne-2-yle, acétyle, benzoyle, tertiobutyle, benzyle et dichlorobenzyle.

6. Procédé suivant la revendication 1 ou la revendication 2, dans lequel $X^2$ est un groupe de formule COX dans laquelle X représente $NH_2$ ou $NHC_nH_{2n+1}$.

7. Procédé suivant l'une quelconque des revendications 1 à 6, dans lequel $R^1$ est le radical méthyle, $R^2$ est l'hydrogène, $R^3$ est le radical méthyle et $R^4$ est l'hydrogène.

8. Procédé suivant la revendication 1, dans lequel le composé préparé est le N - méthyl -

tyrosyl - D - séryl - glycyl - N - méthyl - phénylalanyl - D - sérinamide; le N - méthyl - tyrosyl - D - séryl - glycyl - N - méthyl - phénylalanyl - D - sérin - éthylamide ou la N - méthyl - tyrosyl - D - séryl - glycyl - N - méthyl - phénylalanyl - D - sérine.

9. Procédé suivant la revendication 1 ou la revendication 8, dans lequel le composé de formule I préparé est sous la forme d'un sel d'addition d'acide chlorhydrique, phosphorique, maléique, acétique, citrique, succinique ou malique ou d'un sel de sodium, de potassium, d'ammonium ou d'alkylamine inférieure.

10. Procédé suivant la revendication 3, dans lequel le composé préparé consiste en tertio - butoxycarbonyl - N - méthyl - O - 2,6 - dichlorobenzyl - tyrosyl - O - benzyl - D - séryl - glycyl - N - méthyl - phénylalanyl - O - benzyl - D - séryl - résine de benzhydrylamine; en tertio - butoxycarbonyl - N - méthyl - O - 2,6 - dichlorobenzyl - tyrosyl - O - benzyl - D - séryl - glycyl - N - méthyl - phénylalanyl - O - benzyl - D - séryloxyméthyl - résine de polystyrène ou en tertio - butoxycarbonyl - N - méthyl - O - 2,6 - dichlorobenzyl - tyrosyl - O - benzyl - D - séryl - glycyl - N - méthylphénylalanyl - O - benzyl - D - sérinéthylamide.

**Patentansprüche für die vertragsstaaten BE, CH, DE, FR, IT, LU, NL, SE**

1. Eine Verbindung der allgemeinen Formel

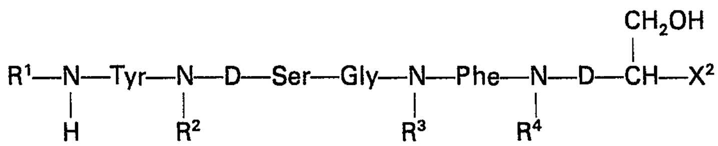

(I)

worin $R^1$ Wasserstoff, Methyl, Äthyl, Propyl, 2-Methyl-2-pentenyl, 2-Methyl-1-pentenyl, Cyclopropylmethyl oder Cyclobutylmethyl bedeutet; $R^2$, $R^3$ und $R^4$ unabhängig voneinander Wasserstoff oder Methyl sind; und $X^2$ —COX ist, wobei X —OH, —$NH_2$, —$NHC_nH_{2n+1}$, worin n 1, 2, 3 oder 4 ist, oder —$OR^5$ darstellt, wobei $R^5$ nied. Alkyl mit 1 bis 4 C-Atomen ist, oder $X^2$ $CH_2OR^6$ ist, wobei $R^6$ Wasserstoff oder nied. Alkyl mit 1 bis 4 C-Atomen bedeutet, oder ein pharmazeutisch annehmbares Salz hievon.

2. Eine Verbindung der Formel (I), wie in Anspruch 1 beanspruch, worin $R^1$ Methyl, $R^2$ Wasserstoff, $R^3$ Methyl und $R^4$ Wasserstoff sind.

3. Eine Verbindung der Formel (I), wie in Anspruch 1 oder Anspruch 2 beansprucht, worin $X^2$ COX bedeutet, wobei X $NH_2$ oder $NHC_nH_{2n+1}$ ist.

4. Eine Verbindung der Formel (I), wie in Anspruch 1 definiert, welche N - Methyl - tyrosyl - D - seryl - glycyl - N - methyl - phenylalanyl - D - serinamid; N - Methyl - tyrosyl - D - seryl - glycyl - N - methyl - phenylalanyl - D - serinäthylamid oder N - Methyltyrosyl - D - seryl - glycyl - N - methyl - phenylalanyl - D - serin ist.

5. Ein Verbindung, wie in einem der Ansprüche 1 bis 4 beansprucht, in Form eines Salz, Phosphor-, Malein-, Essig-, Zitronen-, Bernstein- oder Äpfelsäureadditionssalzes oder eines Natrium-, Kalium, Ammonium- oder nied. Alkylaminsalzes.

6. Eine Verbindung der allgemeinen Formel

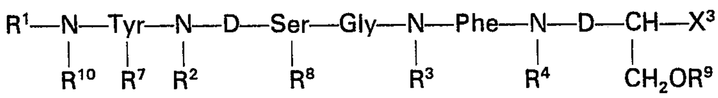

(II)

worin $R^1$, $R^2$, $R^3$ und $R^4$ wie in Anspruch 1 definiert sind, $R^7$ eine Schutzgruppe für das Seitenkettenhydroxyl von Tyrosin darstellt, $R^8$ und $R^9$ Schutzgruppen für das Seitenkettenhydroxyl von Serin sind, $R^{10}$ eine $\alpha$-Aminoschutzgruppe ist und $X^3$ $COX^1$ bedeutet, wobei $X^1$ OH, $NH_2$, $NHC_nH_{2n+1}$, wobei n 1, 2, 3 oder 4 ist, $OR^5$, wobei $R^5$ wie in Anspruch 1 definiert ist, $OR^{11}$, worin $R^{11}$ eine von $R^5$ verschiedene Carboxylschutzgruppe ist, oder einen Festphasenharzträger darstellt, in dem die Brückenbindung zwischen CO und einem Phenyl des Harzträgers die Formel

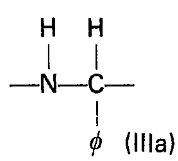

(IIIa)

oder

$-O-CH_2-$

(IIIb)

besitzt, worin $\phi$ Phenyl ist oder $X^3$ $CH_2OR^{12}$ darstellt, wobei $R^{12}$ nied. Alkyl mit 1 bis 4 C-Atomen, eine Hydroxylschutzgruppe, wie durch $R^9$ definiert, oder Wasserstoff ist.

7. Eine Verbindung, wie in Anspruch 6 beansprucht, worin die $\alpha$-Aminoschutzgruppe $R^{10}$ unter

Formyl, Trifluoracetyl, Phthaly, p-Toluolsulfonyl, Nitrophenylsulfenyl, Benzyloxycarbonyl, p-Chlorbenzyloxycarbonyl, p-Nitrobenzyloxycarbonyl, tert.Butyloxycarbonyl, Diisopropylmethoxycarbonyl, Isopropyloxycarbonyl, Cyclopentyloxycarbonyl, Adamantyloxycarbonyl, Cyclohexyloxycarbonyl, Phenylthiocarbonyl, Triphenylmethyl und Trimethylsilyl ausgewählt ist.

8. Eine Verbindung, wie in Anspruch 6 oder Anspruch 7 beansprucht, worin $R^7$, $R^8$ und $R^9$ unabhängig voneinander unter Tosyl, Benzyloxycarbonyl, Tetrahydropyran-2-yl, Acetyl, Benzoyl, tert.Butyl, Benzyl und Dichlorbenzyl ausgewählt sind.

9. Eine Verbindung der Formel (II), wie in Anspruch 6 definiert, welche tert.Butoxycarbonyl - N - methyl - O - 2,6 - dichlorbenzyl - tyrosyl - O - benzyl - D - seryl - glycyl - N - methyl - phenylalanyl - O - benzyl - D - seryl - benzhydrylaminharz; tert.Butoxycarbonyl - N - methyl - O - 2,6 - dichlorbenzyl - tyrosyl - O - benzyl - D - seryl - glycyl - N - methyl - phenylalanyl - O - benzyl - D - seryloxymethyl - polystyrolharz oder tert.Butoxycarbonyl - N - methyl - O - 2,6 - dichlorbenzyl - tyrosyl - O - benzyl - D - seryl - glycyl - N - methyl - phenylalanyl - O - benzyl - D - serinäthylamid ist.

10. Ein Verfahren zur Herstellung einer Verbindung der Formel

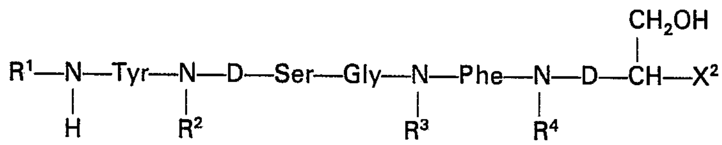

(I)

wie in Anspruch 1 definiert, welches die Entfernung der Schutzgruppen und das Abspalten des Harzträgers, wenn vorhanden, von einer Verbindung der Formel

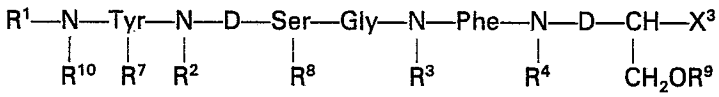

(II)

wie in Anspruch 6 definiert, und, wenn gewünscht, Überführen der erhaltenen Verbindung der Formel (I) in ein pharmazeutisch annehmbares Salz umfaßt.

11. Ein Verfahren, wie in Anspruch 10 beansprucht, welches mit Fluorwasserstoff bewirkt wird.

12. Ein Verfahren, wie in Anspruch 10 oder Anspruch 11 beansprucht, worin $R^{10}$ wie in Anspruch 7 definiert ist und $R^7$, $R^8$ und/oder $R^9$ wie in Anspruch 8 definiert ist/sind.

13. Ein Verfahren zur Herstellung einer Verbindung der Formel (II), wie in Anspruch 6 definiert, welches umfaßt:

(a) Reduzieren einer Verbindung der Formel (II), worin $X^3$ $COX^1$ ist, wobei $X^1$ OH ist, um eine Verbindung der Formel (II) zu erhalten, worin $R^{12}$ Wasserstoff ist, und, wenn gewünscht, Alkylieren des Produktes, um eine Verbindung der Formel (II) zu ergeben, worin $R^{12}$ nied.Alkyl mit 1 bis 4 C-Atomen ist;

(b) aufeinanderfolgendes Koppeln der erforderlichen, geeignet geschützten und/oder aktivierten Aminosäuren unter Festphasensynthesebedingungen an ein Benzhydrylamin-, chlormethyliertes oder Hydroxymethylpolystyrolharz, um eine Verbindung der Formel (II) zu ergeben, worin $X^1$ ein Festphasenpolystyrolharzträger ist, oder

(c) Koppeln der erforderlichen Aminosäuren und

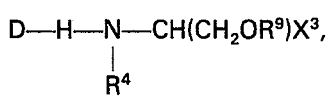

worin $R^4$ und $R^9$ wie in Anspruch 6 definiert sind und $X^3$ $COX^1$ bedeutet, worin $X^1$ $NH_2$, $NHC_nH_{2n+1}$, $OR^5$ oder $OR^{11}$ ist, oder $X^3$ $CH_2OR^{12}$ ist, worin $R^{12}$ von Wasserstoff verschieden ist, jeweils geeignet geschützt und/oder aktiviert, um eine entsprechende Verbindung der Formel (II) zu ergeben, worin $X^3$ wie unmittelbar oben definiert ist.

14. Ein Verfahren, wie in Anspruch 10 beansprucht, worin die hergestellte Verbindung N - Methyl - tyrosyl - D - seryl - glycyl - N - methyl - phenylalanyl - D - serinamid; N - Methyl - tyrosyl - D - serylglycyl - N - methyl - phenylalanyl - D - serinäthylamid oder N - Methyl - tyrosyl - D - seryl - glycyl - N - methyl - phenylalanyl - D - serin ist.

15. Ein Verfahren, wie in Anspruch 13 beansprucht, worin die hergestellte Verbindung tert.Butoxycarbonyl - N - methyl - O - 2,6 - dichlorbenzyl - tyrosyl - O - benzyl - D - seryl - glycyl - N - methyl - phenylalanyl - O - benzyl - D - seryl - benzhydrylaminharz; tert.Butoxycarbonyl - N - methyl - O - 2,6 - dichlorbenzyl - tyrosyl - O - benzyl - D - seryl - glycyl - N - methyl - phenylalanyl - O - benzyl - D - seryloxymethylpolystyrolharz oder tert.Butoxycarbonyl -

N - methyl - O - 2,6 - dichlorbenzyl - tyrosyl - O - benzyl - D - seryl - glycyl - N - methyl - phenylalanyl - O - benzyl - D - serinäthylamid ist.

16. Eine pharmzeutische Zusammensetzung, die eine Verbindung der Formel (I), wie in einem der Ansprüche 1 bis 4 definiert, oder ein pharmazeutisch annehmbares Salz hievon in Verbindung mit einem pharmazeutisch annehmbaren Träger umfaßt.

17. Eine Verbindung der Formel (I), wie in einem der Ansprüche 1 bis 4 beansprucht, oder ein pharmazeutisch annehmbares Salz hievon zur Verwendung als Pharmazeutikum.

**Patentansprüche fur den vertragsstaat AT**

1. Ein Verfahren zur Herstellung einer Verbindung der allgemeinen Formel

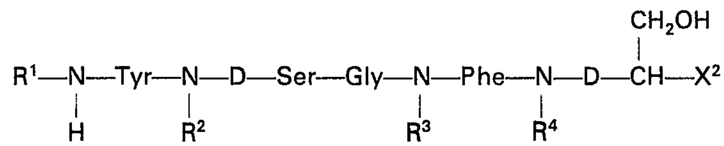

(I)

worin $R^1$ Wasserstoff, Methyl, Äthyl, Propyl, 2-Methyl-2-pentenyl, 2-Methyl-1-pentenyl, Cyclopropylmethyl oder Cyclobutylmethyl ist; $R^2$, $R^3$ und $R^4$ unabhängig voneinander Wasserstoff oder Methyl sind; und $X^2$ —COX ist, wobei X —OH, —$NH_2$, —$NHC_nH_{2n+1}$, worin n 1, 2, 3 oder 4 ist, oder —$OR^5$, worin $R^5$ nied.Alkyl mit 1 bis 4 C-Atomen bedeutet, darstellt, oder $X^2$ $CH_2OR^6$ ist, worin $R^6$ Wasserstoff oder nied.Alkyl mit 1 bis 4 C-Atomen bedeutet, oder eines pharmazeutisch annehmbaren Salzes hievon, welches die Entfernung der Schutzgruppen und die Abspaltung des Harzträgers, wenn vorhanden, von einer Verbindung der allgemeinen Formel

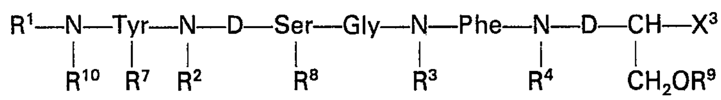

(II)

worin $R^1$, $R^2$, $R^3$ und $R^4$ wie oben definiert sind, $R^7$ eine Schutzgruppe für das Seitenkettenhydroxyl von Tyrosin ist, $R^8$ und $R^9$ Schutzgruppen für das Seitenkettenhydroxyl von Serin darstellen, $R^{10}$ eine $\alpha$-Aminoschutzgruppe darstellt und $X^3$ $COX^1$ ist, wobei $X^1$ OH, $NH_2$, $NHC_nH_{2n+1}$, worin n 1, 2, 3 oder 4 ist, $OR^5$, worin $R^5$ wie oben definiert ist, $OR^{11}$, worin $R^{11}$ eine von $R^5$ verschiedene Carboxylschutzgruppe ist, oder einen Festphasenharzträger darstellt, in welchem die Brückenbindung zwischen CO und einem Phenyl des Harzträgers die Formel

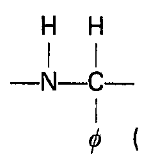

(IIIa) oder

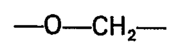

(IIIb)

besitzt, worin $\phi$ Phenyl darstellt oder $X^3$ $CH_2OR^{12}$ bedeutet, wobei $R^{12}$ nied.Alkyl mit 1 bis 4 C-Atomen, eine Hydroxylschutzgruppe, wie durch $R^9$ definiert, oder Wasserstoff ist, und, wenn gewünscht, Überführen der erhaltenen Verbindung der Formel (I) in eine pharmazeutisch annehmbares Salz umfaßt.

2. Verfahren, wie in Anspruch 1 beansprucht, welches mit Fluorwasserstoff bewirkt wird.

3. Verfahren zur Herstellung einer Verbindung der allgemeinen Formel

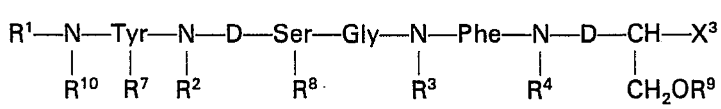

(II)

worin $R^1$, $R^2$, $R^3$ und $R^4$ wie in Anspruch 1 definiert sind, $R^7$ eine Schutzgruppe für das Seitenkettenhydroxyl von Tyrosin ist, $R^8$ und $R^9$ Schutzgruppen für das Seitenkettenhydroxyl von Serin darstellen, $R^{10}$ eine $\alpha$-Aminoschutzgruppe ist und $X^3$ $COX^1$ darstellt, wobei $X^1$ OH, $NH_2$, $NHC_nH_{2n+1}$, worin n 1, 2, 3 oder 4 ist, $OR^5$, wobei $R^5$ wie oben definiert ist, $OR^{11}$, wobei $R^{11}$ eine andere Carboxylschutzgruppe darstellt als $R^5$, oder ein Festphasenharzträger ist, in welchem die Brückenbindung zwischen CO und einem Phenyl des Harzträgers die Formel

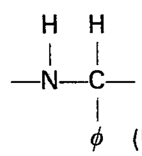

(IIIa) oder

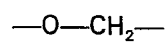

(IIIb)

aufweist, worin $\phi$ Phenyl darstellt, oder $X^3$ $CH_2OR^{12}$ bedeutet, wobei $R^{12}$ nied.Alkyl mit 1 bis 4 C-Atomen, eine Hydroxylschutzgruppe, wie durch $R^9$ definiert, oder Wasserstoff ist, welches umfaßt:

(a) Reduzieren einer Verbindung der Formel (II), worin $X^3$ $COX^1$ bedeutet, wobei $X^1$ OH ist, um eine Verbindung der Formel (II) zu ergeben, worin $R^{12}$ Wasserstoff ist, und, wenn gewünscht, Alkylieren des Produktes, um eine Verbindung der Formel (II) zu ergeben, worin $R^{12}$ nied.Alkyl mit 1 bis 4 C-Atomen ist; oder

(b) aufeinanderfolgendes Koppeln der erforderlichen, geeignet geschützten und/oder aktivierten Aminosäuren unter Festphasensynthesebedingungen an ein Benzhydrylamin-, chlormethyliertes oder Hydroxymethylpolystyrolharz, um eine Verbindung der Formel (II) zu ergeben, worin $X^1$ ein Festphasenpolystyrolharzträger ist, oder

(c) Koppeln der erforderlichen Aminosäuren und

$$\text{D—H—}\underset{\displaystyle R^4}{\underset{|}{\text{N}}}\text{—CH(CH}_2\text{OR}^9\text{)X}^3,$$

worin $R^4$ und $R^9$ wie in Anspruch 1 definiert sind, und $X^3$ $COX^1$ bedeutet, wobei $X^1$ $NH_2$, $NHC_nH_{2n+1}$, $OR^5$ oder $OR^{11}$ ist, oder $X^3$ $CH_2OR^{12}$ darstellt, wobei $R^{12}$ von Wasserstoff verschieden ist, jeweils geeignet geschützt und/oder aktiviert, um eine entsprechende Verbindung der Formel (II) zu ergeben, worin $X^3$ unmittelbar wie oben definiert ist.

4. Ein Verfahren, wie in einem der Ansprüche 1 bis 3 beansprucht, worin die $\alpha$-Aminoschutzgruppe $R^{10}$ unter Formyl, Trifluoracetyl, Phthalyl, p-Toluolsulfonyl, Nitrophenylsulfenyl, Benzyloxycarbonyl, p-Chlorbenzyloxycarbonyl, p-Nitrobenzyloxycarbonyl, tert.Butyloxycarbonyl, Diisopropylmethoxycarbonyl, Isopropyloxycarbonyl, Cyclopentyloxycarbonyl, Adamantyloxycarbonyl, Cyclohexyloxycarbonyl, Phenylthiocarbonyl, Triphenylmethyl und Trimethylsilyl ausgewählt wird.

5. Ein Verfahren, wie in einem der Ansprüche 1 bis 4 beansprucht, worin $R^7$, $R^8$ und $R^9$ unabhängig voneinander unter Tosyl, Benzyloxycarbonyl, Tetrahydropyran-2-yl, Acetyl, Benzoyl, tert.Butyl, Benzyl und Dichlorbenzyl ausgewählt werden.

6. Ein Verfahren, wie in Anspruch 1 oder Anspruch 2 beansprucht, worin $X^2$ COX ist, wobei X $NH_2$ oder $NHC_nH_{2n+1}$ bedeutet.

7. Ein Verfahren, wie in einem der Ansprüche 1 bis 6 beansprucht, worin $R^1$ Methyl, $R^2$ Wasserstoff, $R^3$ Methyl und $R^4$ Wasserstoff bedeuten.

8. Ein Verfahren, wie in Anspruch 1 beansprucht, worin die hergestellte Verbindung N - Methyl - tyrosyl - D - seryl - glycyl - N - methyl - phenylalanyl - D - serinamid; N - Methyl - tyrosyl - D - seryl - glycyl - N - methyl - phenylalanyl - D - serinäthylamid oder N - Methyl - tyrosyl - D - seryl - glycyl - N - methyl - phenylalanyl - D - serin ist.

9. Ein Verfahren, wie in Anspruch 1 oder Anspruch 8 beansprucht, worin die hergestellte Verbindung der Formel (I) in Form eines Salz-, Phosphor-, Malein-, Essig-, Zitronen-, Bernstein- oder Äpfelsäureadditionssalzes oder eines Natrium-, Kalium-, Ammonium- oder nied.Alkylaminsalzes ist.

10. Ein Verfahren, wie in Anspruch 3 beansprucht, worin die hergestellte Verbindung tert.Butoxycarbonyl - N - methyl - O - 2,6 - dichlorbenzyl - tyrosyl - O - benzyl - D - seryl - glycyl - N - methyl - phenylalanyl - O - benzyl - D - seryl - benzhydrylaminharz; tert.Butoxy - carbonyl - N - methyl - O - 2,6 - dichlorbenzyl - tyrosyl - O - benzyl - D - seryl - glycyl - N - methyl - phenylalanyl - O - benzyl - D - seryloxymethylpolystyrolharz oder tert.Butoxycarbonyl - N - methyl - O - 2,6 - dichlorbenzyl - tyrosyl - O - benzyl - D - seryl - glycyl - N - methyl - phenyl - alanyl - O - benzyl - D - serinäthylamid ist.